# EUROPÄISCHE PATENTSCHRIFT

(11) **EP 3 109 026 B1**
(45) Veröffentlichungstag und Bekanntmachung des Hinweises auf die Patenterteilung: **28.11.2018**
(21) Anmeldenummer: 15173459.7
(22) Anmeldetag: 23.06.2015
(51) Int. Cl.: B29C 45/14, B29C 33/12

(54) **VORRICHTUNG ZUR HERSTELLUNG VON KUNSTSTOFFTEILEN MIT EINLEGETEILEN**
DEVICE FOR THE PRODUCTION OF PLASTICS OBJECTS WITH INSERTS
DISPOSITIF DE FABRICATION DE PIÈCES EN MATIÈRE PLASTIQUE AYANT DES INSERTS

(43) Veröffentlichungstag der Anmeldung: 28.12.2016
(73) Patentinhaber: Gerresheimer Regensburg GmbH, 93047 Regensburg (DE)
(72) Erfinder: Hutter, Josef, 94333 Geiselhöring (DE); Wiglenda, Michael, 92699 Irchenrieth (DE); Schlögl, Gerhard, 92536 Pfreimd (DE)
(74) Vertreter: Hannke, Christian

(56) Entgegenhaltungen:
- EP-A1- 2 140 896
- EP-A1- 2 468 209
- EP-A1- 3 127 677
- WO-A1-2012/150897
- WO-A1-2015/033951
- US-A1- 2012 009 816
- US-A1- 2013 138 047

## Beschreibung

Die Erfindung betrifft eine Vorrichtung zur Herstellung von Kunststoffteilen mit jeweils mindestens einem stabförmigen metallischen Einlegeteil, insbesondere medizinische Stechmittel, in einem Spritzgießverfahren, umfassend ein erstes Werkzeughälftenelement und ein zweites Werkzeughälftenelement, wobei zumindest eines der Werkzeughälftenelemente entlang einer Schließrichtung (Z) derart verlagerbar ist, dass die beiden Werkzeughälftenelemente in einer Schließposition zusammen eine abgeschlossene Kavität zum Ausformen eines Kunststoffteils ausbilden, wobei das Einlegeteil, zum Umspritzen mit Kunststoff, zumindest abschnittsweise in die Kavität hineinragend und im Wesentlichen zwischen den beiden Werkzeughälftenelementen in einer Werkzeugtrennebene, welche eine Längsrichtung (X) und eine Breitenrichtung (Y) umfasst, angeordnet ist.
Gattungsgemäße Vorrichtungen zur Herstellung von Kunststoffteilen mit jeweils mindestens einem stabförmigen metallischen Einlegeteil, insbesondere medizinische Stechmittel, in einem Spritzgießverfahren sind aus dem Stand der Technik EP 2140 896, EP 2468 209 sowie EP 3127 667 bekannt. Speziell in der Medizintechnik werden unterschiedlichste Einlegeteile, wie beispielsweise Nadeln, Kanülen oder dergleichen, in Spritzgießwerkzeuge eingelegt, um sie in der durch das Spritzgießwerkzeug ausgestalteten Kavität mit Kunststoff zu umspritzen. In der Regel werden die Einlegeteile in ein unteres Werkzeughälftenelement einer vertikalen Werkzeuganordnung eingelegt. Es ist jedoch auch denkbar, eine horizontale Werkzeuganordnung zu verwenden. Der Kunststoff wird üblicherweise über einen oder mehrere Angusskanäle der Kavität zugeführt und tritt über eine Angussöffnung oder Düse in diese ein. Nach dem Umspritzen des Einlegeteils wird das fertige Erzeugnis, nämlich das Kunststoffteil mit dem Einlegeteil, über Auswurfeinrichtungen aus dem entsprechenden Werkzeughälftenelement ausgeworfen und durch eine Entnahmeeinrichtung aus dem Spritzgießwerkzeug entnommen. Üblicherweise bezeichnet man das Werkzeughälftenelement, welches die Düse umfasst; als Düsenseite und das Werkzeughälftenelement, welches den Auswerfer umfasst, als Auswerferseite.

Auf diese Weise werden Kunststoffteile mit Einlegeteilen speziell für medizinische Anwendungen, beispielsweise Spritzen, Infusionssets, Stechhilfen und Anstechelemente von Kapselinhalatoren, etc., hergestellt. Hierbei ist oftmals eine sehr geringe Toleranz von Nadel bzw. Kanüle zum Kunststoffteil akzeptabel. Außerhalb dieser Toleranz positionierte Einlegeteile können gerade im Einsatz am menschlichen Körper enorme Schmerzen oder schwerwiegende Schäden durch eine ausbleibende Funktion eines medizinischen Geräts hervorrufen. Ferner können derartig fehlerhafte Bauteile medizinische Geräte, welche diese Bauteile verwenden, beschädigen. Demnach sind die Toleranzbereiche bei einem solchen Herstellungsverfahren extrem gering, um die volle Funktionsfähigkeit eines jeden derart hergestellten Kunststoffteils gewährleisten zu können. Bereits geringste Lageabweichungen eines zu umspritzenden Einlegeteils können dazu führen, dass ein medizinisches Kunststoffteil später nicht gebraucht werden kann. Hieraus ergibt sich die Herausforderung, dass die Einlegeteile, welche speziell in der Medizintechnik sehr kleine Ausmaße annehmen können, äußerst präzise in die Kavität des Spritzgießwerkzeuges eingelegt werden müssen.

Bisher wurde versucht, diese geringen Toleranzen durch präzise Handlingroboter zu erreichen. Derartig genaue Handlingssysteme sind jedoch sehr teuer und von der Leistungsfähigkeit kaum im Stande, die geforderten Toleranzen einzuhalten. Zum anderen ist die Gefahr recht hoch, dass die Einlegeteile nach dem korrekten Positionieren nochmals die Position verändern, da das Einlegeteil zwischen der Positionierung und dem Spritzgießprozess nur unzureichend fixiert werden kann. Alternativ werden Einlegteile häufig auf einen Anschlag geschoben. Da die oben erwähnten Gegenstände wie Nadeln und Kanülen jedoch sehr empfindliche Schliffe aufweisen, welche enorm kritisch für die Funktion eines medizinischen Gerätes sind, ist dies hier nicht möglich. So würde der distale Schliff beim Auftreffen auf einen Anschlag ebenso zerstört wie durch einen Schieber der an diesem distalen Ende anschieben soll.

Aufgabe der vorliegenden Erfindung ist es demnach, eine Vorrichtung zur Herstellung von Kunststoffteilen mit jeweils mindestens einem stabförmigen metallischen Einlegeteil, insbesondere medizinische Stechmittel, in einem Spritzgießverfahren bereitzustellen, welche die oben genannten Probleme umgeht.

Diese Aufgabe wird gelöst von einer Vorrichtung zur Herstellung von Kunststoffteilen mit jeweils mindestens einem stabförmigen metallischen Einlegeteil, insbesondere medizinische Stechmittel, in einem Spritzgießverfahren, umfassend ein erstes Werkzeughälftenelement und ein zweites Werkzeughälftenelement, wobei zumindest eines der Werkzeughälftenelemente entlang einer Schließrichtung (Z) derart verlagerbar ist, dass die beiden Werkzeughälftenelemente in einer Schließposition zusammen eine abgeschlossene Kavität zum Ausformen eines Kunststoffteils ausbilden, wobei das Einlegeteil, zum Umspritzen mit Kunststoff, zumindest abschnittsweise in die Kavität hineinragend und im Wesentlichen zwischen den beiden Werkzeughälftenelementen in einer Werkzeugtrennebene, welche eine Längsrichtung (X) und eine Breitenrichtung (Y) umfasst, angeordnet ist. Die Vorrichtung zeichnet sich dadurch aus, dass zumindest eines der Werkzeughälftenelemente zumindest ein Fixiermittel aufweist, mittels welchem das Einlegeteil in der Breitenrichtung (Y) fixierbar ist, und das ein Positioniermittel an einem proximalen Ende des Einlegeteils anordenbar ist, durch welches das in Breitenrichtung (Y) fixierte Einlegeteil entlang der Längsrichtung (X) zu einer Spritzposition verlagerbar ist.

In der erfindungsgemäßen Vorrichtung werden demnach die Einlegeteile vorzugsweise mit einer sehr grob definierten Position in das Werkzeug eingelegt. Diese erste Position weicht dabei bevorzugt in einem erheblichen Maß von der gewünschten axialen Spritzposition ab. Anschließend ist das Einlegeteil an seinem proximalen Ende durch das Positioniermittel zu der Spritzposition verlagerbar. Unter einem proximalen Ende des Einlegeteils ist hierbei das Ende ohne Schliff zu verstehen. Da das Einlegeteil überdies in einer Breitenrichtung (Y) fixiert ist kann das Einlegeteil auf eine einfache Art und Weise sehr exakt in der Kavität positioniert werden. Es ist weder ein komplizierter Handlingroboter notwendig, noch ist es notwendig, dass das Einlegeteil an seinem distalen Ende, d.h. seinem Ende mit Schliff an einem Anschlag anschlägt.

Vorzugsweise weist das Positioniermittel einen Schieberkern auf, welcher im Wesentlichen in der Werkzeugtrennebene angeordnet ist. Dieser Schieberkern weist weiterhin eine Stirnfläche auf, mittels welcher eine Stirnfläche am proximalen Ende des Einlegeteils kontaktierbar ist. Dadurch ist auf das Einlegeteil eine Translationskraft übertragbar und das Einlegeteil ist zu der Spritzposition entlang einer Längsrichtung (X) verlagerbar. Diese Stirnfläche ist bevorzugt eine ebene Fläche. Es sind jedoch anderweitige Ausgestaltungen der Stirnfläche denkbar. Insbesondere für den Fall, dass die zu kontaktierende Stirnfläche am proximalen Ende des Einlegeteils nicht als eine ebene Fläche ausgebildet ist, kann es vorteilhaft sein dass die Stirnfläche des Schieberkerns eine abweichende Form aufweist. Vorstellbar sind beispielsweise Formen komplementär zu der Stirnfläche des Einlegeteils oder Formen, welche als eine anderweitige Aufnahme ausgestaltet ist. Bevorzugt ist der Schieberkern weiterhin stabförmig ausgebildet und weist bevorzugt einen größeren Durchmesser als das Einlegeteil auf. Besonders bevorzugt entspricht der Durchmesser Schieberkerns dem Durchmesser des Einlegeteils.

Gemäß einem vorteilhaften Gedanken der Erfindung umfasst das Positioniermittel ein Endstück, an welchem der Schieberkern angeordnet ist. Es ist denkbar, dass das Endstück und der Schieberkern aus demselben Material, beispielsweise einem hitzebeständigen Kunststoff oder einem Metall bestehen. Es wäre aber auch denkbar, dass das Endstück und der Schieberkern aus unterschiedlichen Materialien bestehen. Vorstellbar wäre außerdem den Schieberkern derart an dem Endstück anzuordnen, dass dieser oder das Endstück ausgewechselt werden können. Besonders bevorzugt weist der Schieberkern einen nagelartigen Endabschnitt mit einem Kopf auf. Vorzugsweise ist dieser Endabschnitt in dem Endstück eingebettet.

Vorzugsweise weisen die Werkzeughälftenelemente Aufnahmen auf, welche abschnittsweise komplementär zu dem Endstück ausgebildet sind. Bevorzugt ist in der Schließposition der Werkzeughälftenelemente das Endstück des Positioniermittels in den Aufnahmen aufgenommen. Vorteilhaft umfassen demnach die Werkzeughälftenelemente in der Schließposition das Endstück zumindest teilweise.

Nach einer weiteren bevorzugten Ausführungsform umfasst zumindest eine der Werkzeughälften eine Betätigungsfläche und das Endstück zumindest eine Rampenfläche. Die Betätigungsfläche beziehungsweise die Rampenfläche sind bevorzugt als Schrägen ausgebildet. Dabei schließen beide Schrägen bevorzugt einen gleichen Winkel (α, β) zu einem Lot ein. Vorzugsweise liegt dieser Winkel (α, β) zwischen 10° und 60°. Bei einer Schließbewegung der Werkzeughälftenelemente ist vorzugsweise das Endstück von einer Ausgangsposition durch ein Aufgleiten der zumindest einen Betätigungsfläche der Werkzeughälften auf die zumindest eine Rampenfläche des Endstücks in eine Endposition verlagerbar. Demnach wird durch die Schließbewegung der Werkzeughälftenelemente eine Verlagerung des Positioniermittels verursacht. Dies stellt eine besonders einfache Ausgestaltung einer Schiebermechanik für den Schieberkern dar. Es sind aber auch anderweitige Schiebermechaniken denkbar, beispielsweise könnte der Schieberkern hydraulisch, pneumatisch, durch Federkraft oder durch ein Getriebe in die Endposition verlagert werden.

Bevorzugt umfasst die Vorrichtung weiterhin eine Rückstelleinrichtung, mittels welcher das Endstück bei einer Öffnungsbewegung der Werkzeughälftenelemente von der Endposition in die Ausgangsposition verlagerbar ist. Eine derartige Rückstelleinrichtung könnte beispielsweise ein Federelement sein. Denkbar wäre allerdings auch eine hydraulisches, pneumatisches oder ein anderweitiges mechanisches Stellglied, um das Endstück beziehungsweise das Positioniermittel wieder in die Ausgangsposition zu bewegen. Es wäre auch vorstellbar, dass in einer der beiden Werkzeughälftenelemente Führungselemente für das Endstück angeordnet sind. Dies könnte beispielsweise durch zumindest eine sich entlang der Längsrichtung (X) erstreckende Ausnehmung am Endstück, welche komplementär zu zumindest einem Führungsvorsprung ist an einer der Werkzeughälftenelemente ausgestaltet sein. Vorstellbar wäre auch das zumindest eines der Werkzeughälftenelemente die besagte zumindest eine Ausnehmung aufweist und der zumindest eine Führungsvorsprung an dem Endstück angeordnet ist.

Gemäß einer bevorzugten Ausführungsform ist die Spritzposition des Einlegeteils durch die Endposition des Endstücks definiert, in der eine Stirnfläche des Endstücks die jeweiligen Stirnfläche der Aufnahmen der Werkzeughälften kontaktiert, und die Länge des Schieberkerns festgelegt. Das zu umspritzende Einlegeteil wird demnach zunächst zwischen die Werkzeughälftenelemente eingebracht, wobei diese erste Position bevorzugt in einem erheblichen Maß von der gewünschten axialen Spritzposition abweicht. Vorzugsweise wird das Einlegeteil mittels des Schieberkerns zu der gewünschten Spritzposition geschoben. Das Einlegeteil kann somit sehr exakt positioniert werden. Für den Fall, dass eine Anpassung der Spritzposition erforderlich ist, da beispielsweise ein kürzeres oder längeres Einlegeteil verwendet werden soll, muss lediglich das Positioniermittel bzw. der Schieberkern ausgetauscht werden.

Gemäß einer weiteren bevorzugten Ausführungsform weist das erste Werkzeughälftenelement ein erstes Fixiermittel und das zweite Werkzeughälftenelement ein zweites Fixiermittel auf. Diese Fixiermittel münden bevorzugt in die Werkzeugtrennebene und sind weiter bevorzugt außerhalb der Kavität angeordnet. Vorteilhafterweise wird durch diese beiden Fixiermittel das Einlegeteil in der Breitenrichtung (Y) der Werkzeughälftenelemente fixiert. Das Einlegeteil ist somit lediglich entlang der Längsrichtung (X) verlagerbar.

Bevorzugt umfasst das erste Fixiermittel einen magnetischen Abschnitt zum fixieren des Einlegeteils, wobei der magnetische Abschnitt flächig mit einer Oberfläche des ersten Werkzeughälftenelement abschließt. Es wäre auch denkbar, dass anstatt der magnetischen Wirkung ein anderweitiger Ladungseffekt zum Fixieren des Einlegeteils genutzt wird.

Vorzugsweise umfasst das zweite Fixiermittel zumindest einen Zentrierstift. Dieser Zentrierstift ist bevorzugt durch ein Federelement druckbeaufschlagt, so dass er in einer offenen Position der Werkzeughälftenelemente abschnittsweise in die Werkzeugtrennebene hineinragend ist. In der Schließposition der Werkzeughälftenelemente ist der Zentrierstift gegen die Federkraft aus der Werkzeugtrennebene entgegen der Schließrichtung (Z) verlagerbar. Bevorzugt umfasst das Federelement eine Spiralfeder. Denkbar sind aber auch anderweitige Federarten wie beispielsweise Schenkelfedern oder Torsionsfedern. Ferner wäre vorstellbar, das Federelement als ein Elastomer auszubilden.

Besonders bevorzugt weist der Zentrierstift an seiner Stirnseite Zentrierflächen auf, um das Einlegeteil bezüglich der Breitenrichtung (Y) zu zentrieren. Diese Zentrierflächen sind vorzugsweise kreisbogenförmig oder konisch ausgebildet. Es sind allerdings auch anderweitige Formen vorstellbar beispielsweise teilweise komplementär zu dem Einleigeteil ausgebildete Formen. Wird das Einlegeteil lediglich unter Verwendung einer magnetischen (bzw. Nutzung anderweitiger Ladungseffekte) Fixierung gehalten, so ist bei einer Verlagerung des Einlegeteils in Längsrichtung (X) durch das Positioniermittel ein Verspringen oder ein ungewünschtes Verrücken des Einlegeteils möglich. Durch einen zusätzlichen Zentrierstift wird dies sehr effizient verhindert.

Gemäß einer bevorzugten Ausführungsform enthält die Vorrichtung zumindest einen zu der Kavität führenden Angusskanal, welcher partiell in dem zweiten Werkzeughälftenelement geführt ist. Da das Einlegeteil und damit auch das korrespondierende Positioniermittel, insbesondere der Schieberkern und die Schiebermechanik vorzugsweise in etwa in der Werkzeugtrennebene liegen, kann dies zur Folge haben, dass gerade bei höher belegten Werkzeugen der Schiebermechanismus mit einem Angusskanal in Konflikt geraten könnte. Um dies zu verhindern, wird vorteilhaft der Angusskanal partiell in der Düsenseite des Werkzeuges geführt.

Nach einer weiteren bevorzugten Ausführungsform ist eine vorzugsweise trapezförmige Schieberkernabdeckung im Bereich des zumindest einen Angusskanals zwischen dem Schieberkern und dem Angusskanal angeordnet. Dies soll verhindern, dass die Schiebermechanik durch den Anguss verdreckt oder gar Partikel aus dem Anguss in die Kavität gelangen.

Vorzugsweise sind das erste und das zweite Werkzeughälftenelement horizontal angeordnet sind, wobei das zweite Werkzeughälftenelement in vertikaler Schließrichtung (Z) oberhalb des ersten Werkzeughälftenelements angeordnet ist.

Weitere Vorteile, Ziele und Eigenschaften der vorliegenden Erfindung werden anhand nachfolgender Beschreibung der anliegenden Figuren erläutert. Gleichartige Komponenten können in den verschiedenen Ausführungsformen gleiche Bezugszeichen aufweisen.

In den Figuren zeigen:
- Fig.1: eine Schnittdarstellung Vorrichtung zur Herstellung von Kunststoffteilen mit jeweils mindestens einem Einlegeteil in einer Offenposition;
- Fig.2: eine Schnittdarstellung Vorrichtung zur Herstellung von Kunststoffteilen mit jeweils mindestens einem Einlegeteil in einer Schließposition;
- Fig.3: eine Schnittdarstellung eines Fixiermittels in der Vorrichtung zur Herstellung von Kunststoffteilen mit jeweils mindestens einem Einlegeteil;
- Fig.4: eine Schnittdarstellung eines Fixiermittels in der Vorrichtung zur Herstellung von Kunststoffteilen mit jeweils mindestens einem Einlegeteil;
- Fig.5: eine Schnittdarstellung eines Kunststoffteils mit einem Einlegeteil;
- Fig.6: isometrische Ansicht eines Schieberkerns mit Schieberkernabdeckung.

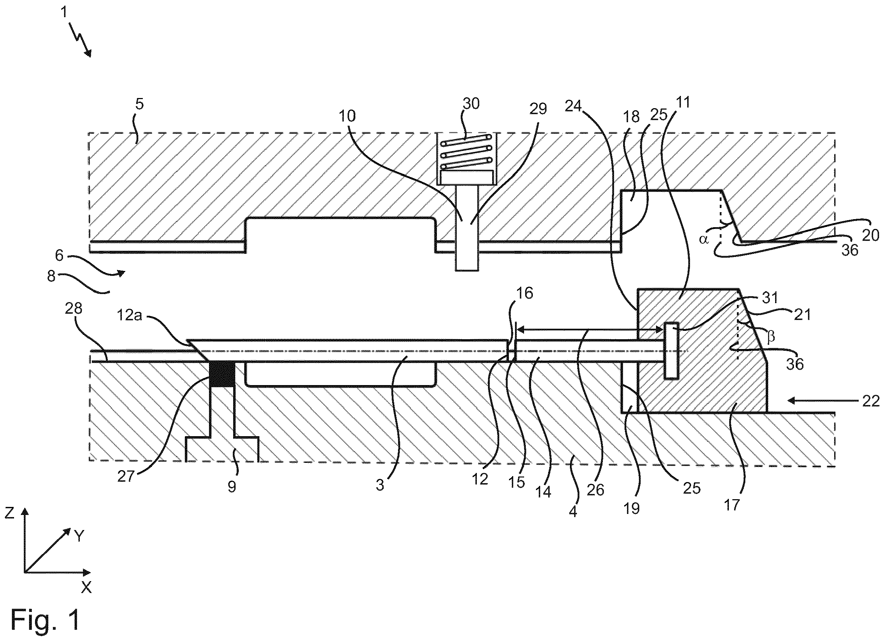

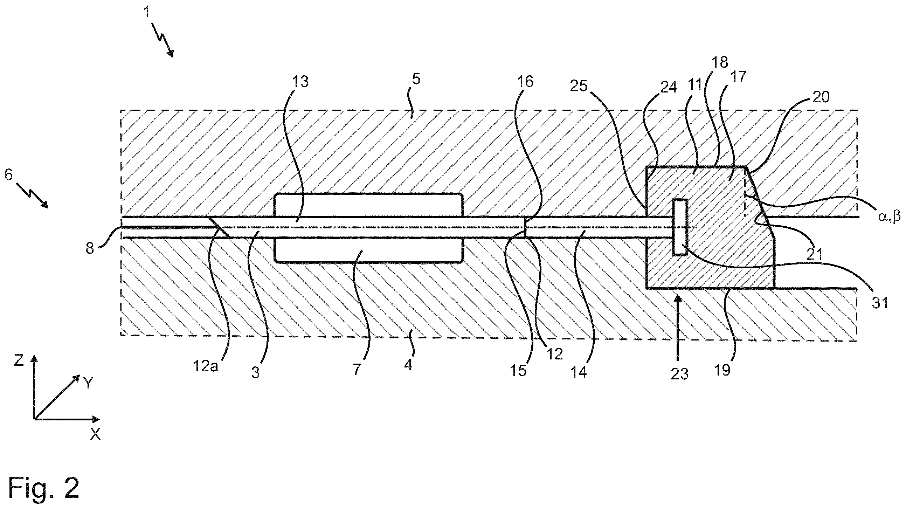

Fig. 1 und Fig. 2 zeigen eine Vorrichtung (1) zur Herstellung von Kunststoffteilen (2) mit jeweils mindestens einem stabförmigen metallischen Einlegeteil (3), insbesondere medizinisehe Stechmittel, in einem Spritzgießverfahren. Fig. 1 zeigt dabei eine Offenposition (6a) und Fig. 2 eine Schließposition (6b) der Vorrichtung.

Die Vorrichtung (1) umfasst ein erstes Werkzeughälftenelement (4) und ein zweites Werkzeughälftenelement (5). Zumindest eines der Werkzeughälftenelemente (4, 5) ist entlang einer Schließrichtung (Z) derart verlagerbar, dass die beiden Werkzeughälftenelemente (4, 5) in einer Schließposition (6b) zusammen eine abgeschlossene Kavität (7) zum Ausformen eines Kunststoffteils (2) ausbilden. Die beiden Werkzeughälftenelemente (4, 5) können sich dabei in einer horizontalen Richtung erstrecken, wobei die Schließrichtung (Z) dann eine vertikale Richtung wäre. Das zweite Werkzeughälftenelement (5) wäre somit in vertikaler Schließrichtung (Z) oberhalb des ersten Werkzeughälftenelements (4) angeordnet. Ebenso wäre es aber möglich, dass die beiden Werkzeughälftenelemente (4, 5) sich entlang einer vertikalen Richtung erstrecken, wobei die Schließrichtung (Z) dann eine horizontale Richtung wäre.

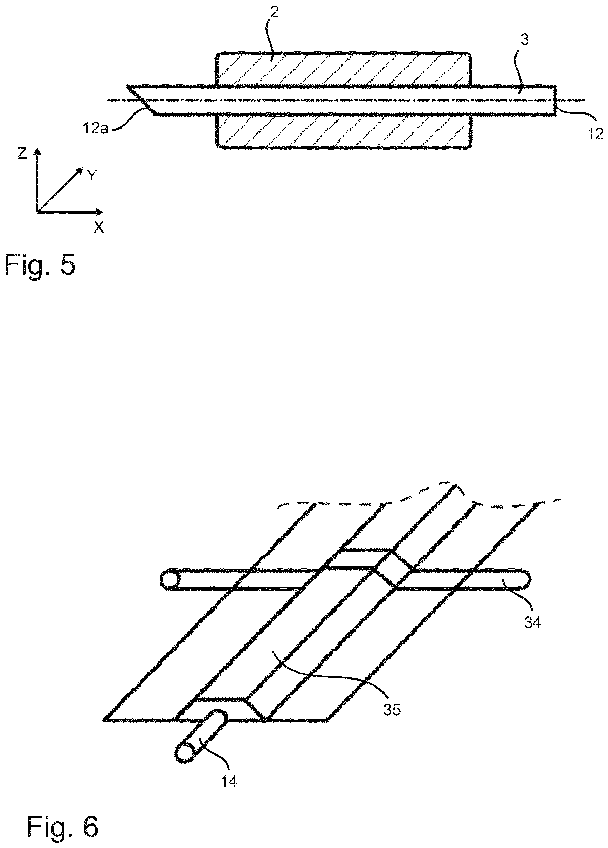

Das Einlegeteil (3) zum Umspritzen mit Kunststoff ragt zumindest abschnittsweise in die Kavität (7) und ist im Wesentlichen zwischen den beiden Werkzeughälftenelementen (4, 5) in einer Werkzeugtrennebene (8) angeordnet. Diese Werkzeugtrennebene (8) umfasst eine Längsrichtung (X) und eine Breitenrichtung (Y). In diesem Ausführungsbeispiel ist die Kavität (7) zylinderförmig ausgestaltet und das Einlegeteil (3) ragt entlang der Längsrichtung (X) beidseitig über die Kavität (7) hinaus. Das Einlegeteil (3) besitzt ein proximales (12) und ein distales Ende (12a), wobei das distale Ende(12a) mit einem Schliff ausgestattet ist. Ein beispielhaftes mit der Kavität (7) geformtes Kunststoffteil (2) mit einem stabförmigen metallischen Einlegeteil (3) ist in Fig. 5 gezeigt.

Die beiden Werkzeughälftenelemente (4, 5) weisen weiterhin jeweils ein Fixiermittel (9, 10) auf, mittels welchem das Einlegeteil (3) in der Breitenrichtung (Y) fixierbar ist. Weiterhin ist ein Positioniermittel (11) an einem proximalen Ende (12) des Einlegeteils (3) anordenbar, durch welches das in Breitenrichtung (Y) fixierte Einlegeteil (3) entlang der Längsrichtung (X) zu einer Spritzposition (13) verlagerbar ist. Das Positioniermittel (11) umfasst einen Schieberkern (14), welcher im Wesentlichen in der Werkzeugtrennebene (8) angeordnet ist. Dieser Schieberkern (14) besitzt wiederum eine Stirnfläche (15), mittels welcher eine Stirnfläche (16) am proximalen Ende (12) des Einlegeteils (3) kontaktierbar ist. Dadurch ist eine Translationskraft auf das Einlegeteil (3) übertragbar, wodurch das Einlegeteil (3) zu der Spritzposition (13) entlang einer Längsrichtung (X) verlagerbar ist.

Darüber hinaus umfasst das Positioniermittel (11) ein Endstück (17), an welchem der Schieberkern (14) angeordnet ist. Die Werkzeughälftenelemente (4, 5) weisen jeweils eine Aufnahme (18, 19) auf, welche abschnittsweise komplementär zu dem Endstück (17) ausgebildet ist. In der in Fig. 2 gezeigten Schließposition (6b) der Werkzeughälftenelemente (4, 5) ist das Endstück (17) demnach in den Aufnahmen (18, 19) aufgenommen.

Der Schieberkern (14) ist stabförmig ausgebildet und weist einen nagelartigen Endabschnitt (31) mit einem Kopf auf. Dabei ist dieser nagelartige Endabschnitt (31) in dem Endstück (17) eingebettet. Die Aufnahmen (18, 19) der Werkzeughälftenelemente (4, 5) sind derart ausgestaltet, dass der Schieberkern (14) in der Werkzeugtrennebene (8) verlagerbar ist. Insbesondere weisen die Aufnahmen (18, 19) eine gleiche Höhe in Schließrichtung (Z) auf, wodurch der in der Werkzeugtrennebene (8) verlagerbare Schieberkern (14) mittig an dem Endstück (17) angeordnet ist.

Ferner umfasst die zweite Werkzeughälfte (5) eine Betätigungsfläche (20) und das Endstück (17) eine Rampenfläche (21). Die Betätigungsfläche (20) beziehungsweise die Rampenfläche (21) sind als Schrägen ausgebildet. Dabei schließen beide Schrägen bevorzugt einen gleichen Winkel (α, β) zu einem Lot (36) ein. Vorzugsweise liegt dieser Winkel (α, β) zwischen 10° und 60°. Das Endstück (17) ist somit bei einer Schließbewegung der Werkzeughälftenelemente (4, 5) von einer Ausgangsposition (22) durch ein Aufgleiten der Betätigungsfläche (20) auf die Rampenfläche (21) in eine Endposition (23) verlagerbar. Das Endstück beziehungsweise das Positioniermittel wird somit von dem zweiten Werkzeughälftenelement (5) mechanisch betätigt.

In dieser Endposition (23) kontaktiert eine Stirnfläche (24) des Endstücks (17) die jeweiligen Stirnfläche (25) der Aufnahmen (18, 19) der Werkzeughälftenelemente (4, 5). Die Spritzposition (13) des Einlegeteils (3) wird somit durch die Endposition (23) des Endstücks (17) und die Länge (26) des Schieberkerns (14) festgelegt. Für den Fall, dass eine andere Spritzposition (13) des Einlegeteils (3) gewünscht ist, kann das Positioniermittel, beziehungsweise der Schieberkern entsprechend ausgetauscht werden. Weiterhin ist es vorteilhaft, dass die Vorrichtung (1) einen Rückstelleinrichtung umfasst, mittels welcher das Endstück (17) bei einer Öffnungsbewegung der Werkzeughälftenelemente (4, 5) von der Endposition (23) in die Ausgangsposition (22) verlagerbar ist. Eine solche Rückstelleinrichtung ist in den Figuren jedoch nicht gezeigt.

Das erste (4) und das zweite Werkzeughälftenelement (5) weisen weiterhin ein erstes (9) und ein zweites Fixiermittel (10) auf. Beide Fixiermittel (9, 10) münden in die Werkzeugtrennebene (8) und sind außerhalb der Kavität (7) angeordnet. Das Einlegeteil (3) wird demnach durch die beiden Fixiermittel (10) in der Breitenrichtung (Y) der Werkzeughälftenelemente (4, 5) fixiert.

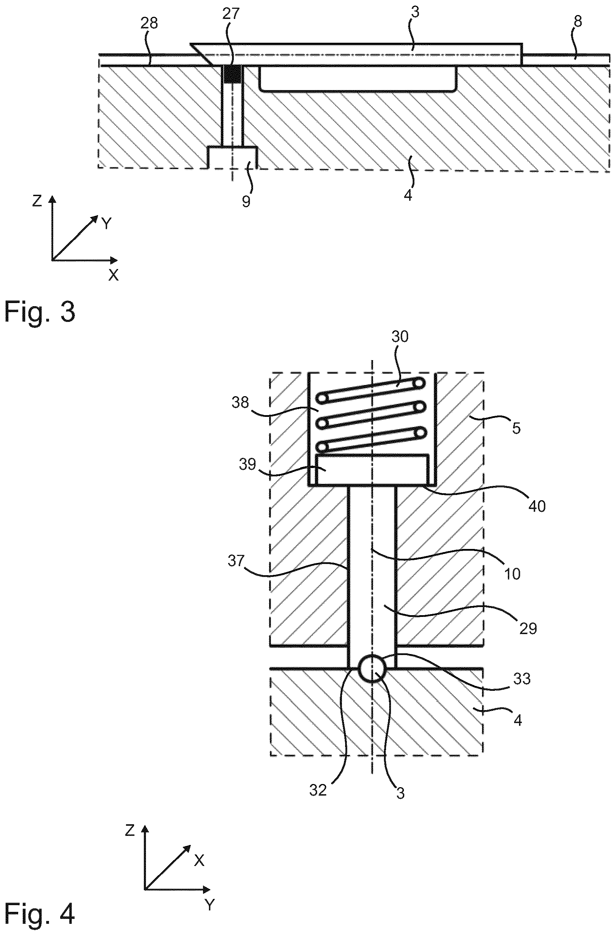

Das erste Fixiermittel (9) umfasst überdies einen magnetischen Abschnitt (27). Der magnetische Abschnitt (27) schließt dabei flächig mit einer Oberfläche (28) des ersten Werkzeughälftenelements (4) ab. Die metallischen Einlegeteile (3) können somit durch die magnetische Kraft des Abschnitts (27) fixiert werden. Eine detaillierte Ansicht des ersten Fixiermittels (9) ist in Fig. 3 dargestellt.

Das zweite Fixiermittel (10) umfasst einen Zentrierstift (29), zum Fixieren und Zentrieren des Einlegeteils (3). Das zweite Fixiermittel (10) ist detailliert in Fig. 4 dargestellt. Der Zentrierstift ist beweglich in einem Kanal (37) des zweiten Werkzeughälftenelements (5) angeordnet. Der Kanal mündet in einem Federraum (38), welcher einen größeren Durchmesser als der Kanal besitzt. In dem Federraum (38) ist eine kolbenartige Platte (39) angeordnet, welche durch ein in dem Federraum angeordnetes Federelement (30) druckbeaufschlagt ist. Das Federelement (30) ist als eine Spiralfeder, welche einen Hub in Schließrichtung (Z) aufweist ausgebildet. Die Mittelachse der Spiralfeder liegt auf einer Mittelachse (41) des Zentrierstifts (29). Der Zentrierstift (29) ist rückseitig mit der kolbenartigen Platte (39) verbunden und somit rückseitig durch das Federelement (30) druckbeaufschlagt. In der Offenposition (6a) der Werkzeughälftenelemente (4, 5) ist die kolbenartige Platte (39) durch die Federkraft des Federelements (30) an einer unteren Innenwand (40) des Federraums (38) anliegend. Demzufolge ist der Zentrierstift (29) durch die Federkraft des Federelements (30) abschnittsweise in die Werkzeugtrennebene (8) hineinragend. Durch das Anliegen der kolbenartigen Platte (39) an der unteren Innenwand (40) des Federraums (38) wird demnach eine maximale in die Werkzeugtrennebene (8) hineinragende Länge des Zentrierstifts (29) festgelegt. Kontaktiert der Zentrierstift (29) bei einer Schließbewegung der Werkzeughälftenelemente (4, 5) das Einlegeteil (3) wird er fortan bei einer weiteren Schließbewegung in den Kanal (37) entgegen der Schließrichtung (Z) verlagert. Dadurch wird wiederum die kolbenartige Platte (39) verlagert und das Federelement (30) gespannt.

Der Zentrierstift (29) weist weiterhin an seiner Stirnseite (32) Zentrierflächen (33) auf, um das Einlegeteil (3) bezüglich der Breitenrichtung (Y) zu zentrieren. In der Regel weisen die Einlegeteile (3) einen kreisförmigen Querschnitt auf. In diesem Ausführungsbeispiel sind Zentrierflächen (33) kreisbogenförmig ausgebildet, wodurch das Einlegeteil zentriert und in Breitenrichtung (Z) fixiert ist.

Die Vorrichtung (1) enthält zumindest einen zu der Kavität (7) führenden Angusskanal (34) welcher zumindest partiell in dem zweiten Werkzeughälftenelement (5) geführt ist. In Fig. 6 wird der Schieberkern (14) dargestellt. Im Bereich des Angusskanals (34) ist zwischen dem Schieberkern (14) und dem Angusskanal (34) eine trapezförmige Schieberkernabdeckung (35) angeordnet. Dies soll verhindern, dass die Schiebermechanik durch den Anguss verdreckt oder gar Partikel aus dem Anguss in die Kavität (7) gelangen.

Im Folgenden soll ein Ablauf zur Herstellung eines Kunststoffteils (2) mit jeweils mindestens einem stabförmigen metallischen Einlegeteil (3) mit der oben beschriebenen Vorrichtung (1) beschrieben werden. Die Werkzeughälftenelemente (4, 5) befinden sich zunächst in einer Offenposition (12a). Die Einlegeteile (3) werden mit einer sehr grob definierten Position zwischen die Werkzeughälftenelemente (4, 5) eingelegt, wobei das proximale Ende (12) (das Ende ohne Schliff) des Einlegeteils (3) zu dem Schieberkern (14) hinweist. Das Einlegeteil (3) ist zunächst nur durch das erste Fixiermittel (9) durch die magnetische Kraft des magnetischen Abschnitts (27) fixiert. Diese erste Position des Einlegeteils (3) weicht dabei in einem erheblichen Maß von der gewünschten axialen Spritzposition (13) ab. In Fig. 1 ist diese Offenposition (12a) Werkzeughälftenelemente (4, 5) dargestellt. Die Stirnfläche (15) des Schieberkerns (14) kontaktiert dabei nicht die Stirnfläche (16) am proximalen Ende (12) des Einlegeteils (3). Das Endstück (17) befindet sich in einer Ausgangsposition (22). Bei einer Schließbewegung bewegen sie die Werkzeughälftenelemente (4, 5) aufeinander zu. Der in die Werkzeugtrennebene hineinragende Zentrierstift (29) kommt nun in Kontakt mit dem Einlegeteil (3). Durch die Zentrierflächen (33) an der Stirnseite (32) des Zentrierstifts (29) wird das Einlegeteil (3) in der Breitenrichtung (Y) zentriert. Dadurch wird ein Verspringen, das vor allem bei Verwendung einer rein magnetischen Fixierung erfolgen kann, oder ungewünschtes Verrücken der Nadel sehr effizient verhindert. Gleichzeitig kontaktieren die Betätigungsfläche (20) des zweiten Werkzeughälftenelements (5) und die Rampenfläche (21) des Endstücks (17). Durch ein Aufgleiten der Betätigungsfläche (20) auf die Rampenfläche (21) wird das Endstück aus seiner Ausgangsposition (22) verlagert. Demzufolge wird auch der Schieberkern (14) zu dem Einlegeteil (3) hin verschoben. Kontaktieren sich im Folgenden die Stirnfläche (15) des Schieberkerns (14) und die Stirnfläche (16) am proximalen Ende (12) des Einlegeteils (3), wird eine Translationskraft auf das Einlegeteil (3) übertragen und das Einlegeteil (3) wird entlang der Längsrichtung (X) verschoben. In einer Schließposition (6b), dargestellt in Fig. 2, befindet sich das Endstück (17) in seiner Endposition (23), in der die Stirnfläche (24) des Endstücks (17) an den jeweiligen Stirnflächen (25) der Aufnahmen (18, 19) der Werkzeughälftenelemente (4, 5) anliegt. Die Werkzeughälftenelemente (4, 5) bilden zusammen eine abgeschlossene Kavität (7) zum Ausformen eines Kunststoffteils (2) aus. Das Einlegeteil (3) befindet sich in der Spritzposition (13). In dieser Position wird es in Längsrichtung (X) von dem Schieberkern (14) begrenzt und in Breitenrichtung von dem ersten und zweiten Fixiermittel fixiert und zentriert. Es ist somit eine sehr genaue Positionierung des Einlegeteils (3) gewährleistet. In dieser Spritzposition (13) erfolgt nun der Spritzvorgang. Daraufhin werden die Werkzeughälftenelemente (4, 5) geöffnet und das fertige Kunststoffteil (2) mit mindestens einem stabförmigen metallischen Einlegeteil (3) kann entnommen werden.

Sämtliche in den Anmeldungsunterlagen offenbarten Merkmale werden als erfindungswesentlich beansprucht, sofern sie einzeln oder in Kombination gegenüber dem Stand der Technik neu sind.

### Bezugszeichenliste

- 1: Vorrichtung zur Herstellung von Kunststoffteilen
- 2: Kunststoffteil
- 3: Einlegeteil
- 3a: erstes Werkzeughälftenelement
- 3b: zweites Werkzeughälftenelement
- 4: distales Ende des Spritzenkörpers
- 5: zylindrisches Endstück
- 6a: Offenposition
- 6b: Schließposition
- 7: Kavität
- 8: Werkzeugtrennebene
- 9: erstes Fixiermittel
- 10: zweites Fixiermittel
- 11: Positioniermittel
- 12: proximales Ende des Einlegeteils
- 12a: distales Ende des Einlegeteils
- 13: Spritzposition
- 14: Schieberkern
- 15: Stirnfläche des Schieberkerns
- 16: Stirnfläche des Einlegeteils
- 17: Endstück
- 18: Aufnahme des ersten Werkzeughälftenelements
- 19: Aufnahme des zweiten Werkzeughälftenelements
- 20: Betätigungsfläche
- 21: Rampenfläche
- 22: Ausgangsposition des Endstücks
- 23: Endposition des Endstücks
- 24: Stirnfläche des Endstücks
- 25: jeweilige Stirnfläche der Aufnahmen
- 26: Länge des Schieberkerns
- 27: magnetischen Abschnitt des ersten Fixiermittels
- 28: Oberfläche (28) des ersten Werkzeughälftenelements
- 29: Zentrierstift
- 30: Federelement
- 31: nagelartiger Endabschnitt des Schieberkerns
- 32: Stirnseite des Zentrierstifts
- 33: Zentrierflächen
- 34: Angusskanal
- 35: Schieberkernabdeckung
- 36: Lot
- 37: Kanal
- 38: Federraum
- 39: kolbenartige Platte
- 40: untere Innenwand des Federraums
- 41: Mittelachse des Zentrierstifts
- X: Längsrichtung
- Y: Breitenrichtung
- Z: Schließrichtung

## Patentansprüche

1. Vorrichtung (1) zur Herstellung von Kunststoffteilen (2) mit jeweils mindestens einem stabförmigen metallischen Einlegeteil (3), welches ein proximales Ende (12) und ein distales Ende (12a) mit einem Schliff aufweist, insbesondere medizinische Stechmittel, in einem Spritzgießverfahren, umfassend ein erstes Werkzeughälftenelement (4) und ein zweites Werkzeughälftenelement (5), wobei zumindest eines der Werkzeughälftenelemente (4, 5) entlang einer Schließrichtung (Z) derart verlagerbar ist, dass die beiden Werkzeughälftenelemente (4, 5) in einer Schließposition (6b) zusammen eine abgeschlossene Kavität (7) zum Ausformen eines Kunststoffteils (2) ausbilden, wobei das Einlegeteil (3), zum Umspritzen mit Kunststoff, zumindest abschnittsweise in die Kavität (7) hineinragend und im Wesentlichen zwischen den beiden Werkzeughälftenelementen (4, 5) in einer Werkzeugtrennebene (8), welche eine Längsrichtung (X) und eine Breitenrichtung (Y) umfasst, angeordnet ist,
**dadurch gekennzeichnet, dass**
zumindest eines der Werkzeughälftenelemente (4, 5) zumindest ein Fixiermittel (9, 10) aufweist, mittels welchem das Einlegeteil (3) in der Breitenrichtung (Y) fixierbar ist, und das ein Positioniermittel (11) an dem proximalen Ende (12) des Einlegeteils (3) anordenbar ist, durch welches das in Breitenrichtung (Y) fixierte Einlegeteil (3) entlang der Längsrichtung (X) zu einer Spritzposition (13) verlagerbar ist.

2. Vorrichtung (1) nach Anspruch 1,
**dadurch gekennzeichnet, dass**
das Positioniermittel (11) einen Schieberkern (14) umfasst, welcher im Wesentlichen in der Werkzeugtrennebene (8) angeordnet ist, wobei der Schieberkern (14) eine Stirnfläche (15) aufweist, mittels welcher eine Stirnfläche (16) am proximalen Ende (12) des Einlegeteils (3) kontaktierbar ist wodurch auf das Einlegeteil (3) eine Translationskraft übertragbar ist und das Einlegeteil (3) zu der Spritzposition (13) entlang einer Längsrichtung (X) verlagerbar ist.

3. Vorrichtung (1) nach Anspruch 1 oder 2,
**dadurch gekennzeichnet, dass**
das Positioniermittel (11) ein Endstück (17), an welchem der Schieberkern (14) angeordnet ist umfasst, wobei die Werkzeughälftenelemente (4, 5) Aufnahmen (18, 19) aufweisen, welche abschnittsweise komplementär zu dem Endstück (17) ausgebildet sind und wobei in der Schließposition (6b) der Werkzeughälftenelemente (4, 5) das Endstück (17) in den Aufnahmen (18, 19) aufgenommen ist.

4. Vorrichtung (1) nach Anspruch 3,
**dadurch gekennzeichnet, dass**
zumindest eine der Werkzeughälften (4, 5) eine Betätigungsfläche (20) umfasst und das Endstück (17) zumindest eine Rampenfläche (21), wobei das Endstück (17) bei einer Schließbewegung der Werkzeughälftenelemente (4, 5) von einer Ausgangsposition (22) durch ein Aufgleiten der zumindest einen Betätigungsfläche (20) auf die zumindest eine Rampenfläche (21) in eine Endposition (23) verlagerbar ist.

5. Vorrichtung (1) nach Anspruch 3 oder 4,
**dadurch gekennzeichnet, dass**
die Vorrichtung (1) eine Rückstelleinrichtung umfasst, mittels welcher das Endstück (17) bei einer Öffnungsbewegung der Werkzeughälftenelemente (4, 5) von der Endposition (23) in die Ausgangsposition (22) verlagerbar ist.

6. Vorrichtung (1) nach Anspruch 4 oder 5,
**dadurch gekennzeichnet, dass**
die Spritzposition (13) des Einlegeteils (3) durch die Endposition (23) des Endstücks (17), in der eine Stirnfläche (24) des Endstücks (17) die jeweiligen Stirnflächen (25) der Aufnahmen (18, 19) der Werkzeughälftenelemente (4, 5) kontaktiert, und die Länge (26) des Schieberkerns (14) festgelegt ist.

7. Vorrichtung (1) nach einem der vorhergehenden Ansprüche,
**dadurch gekennzeichnet, dass**
das erste Werkzeughälftenelement (4) ein erstes Fixiermittel (9) und an das zweite Werkzeughälftenelement (5) ein zweites Fixiermittel (10) aufweist, wobei die Fixiermittel (9, 10) in die Werkzeugtrennebene (8) münden, außerhalb der Kavität (7) angeordnet sind und das Einlegeteil (3) in der Breitenrichtung (Y) der Werkzeughälftenelemente (4, 5) fixieren.

8. Vorrichtung (1) nach Anspruch 7,
**dadurch gekennzeichnet, dass**
das erste Fixiermittel (9) einen magnetischen Abschnitt (27) zum Fixieren des Einlegeteils (3) umfasst, wobei der magnetische Abschnitt (27) flächig mit einer Oberfläche (28) des ersten Werkzeughälftenelements (4) abschließt.

9. Vorrichtung (1) nach Anspruch 7,
**dadurch gekennzeichnet, dass**
das zweite Fixiermittel (10) als zumindest einen Zentrierstift (29) umfasst, zum Fixieren und Zentrieren des Einlegeteils (3), wobei der Zentrierstift (29) durch ein Federelement (30) druckbeaufschlagt ist, so dass er in einer Offenposition (6a) der Werkzeughälftenelemente (4, 5) abschnittsweise in die Werkzeugtrennebene (8) hineinragend ist und in der Schließposition (6b) der Werkzeughälftenelemente (4, 5) gegen die Federkraft aus der Werkzeugtrennebene (8) entgegen der Schließrichtung (Z) verlagerbar ist.

10. Vorrichtung (1) nach Anspruch 9,
**dadurch gekennzeichnet, dass**
der Zentrierstift (29) an seiner Stirnseite (32) Zentrierflächen (33) aufweist, um das Einlegeteil (3) bezüglich der Breitenrichtung (Y) zu zentrieren.

11. Vorrichtung (1) nach einem der vorhergehenden Ansprüche,
**dadurch gekennzeichnet, dass**
die Vorrichtung (1) zumindest einen zu der Kavität (7) führenden Angusskanal (34) enthält, welcher zumindest partiell in dem zweiten Werkzeughälftenelement (5) geführt ist.

12. Vorrichtung (1) nach Anspruch 11,
**dadurch gekennzeichnet, dass**
eine vorzugsweise trapezförmige Schieberkernabdeckung (35) im Bereich des zumindest einen Angusskanals (34) zwischen dem Schieberkern (14) und dem Angusskanal (34) angeordnet ist.

13. Vorrichtung (1) nach einem der vorhergehenden Ansprüche,
**dadurch gekennzeichnet, dass**
das erste (4) und das zweite Werkzeughälftenelement (5) horizontal angeordnet sind, wobei das zweite Werkzeughälftenelement (5) in vertikaler Schließrichtung (Z) oberhalb des ersten Werkzeughälftenelements (4) angeordnet ist.

## Claims

1. Device (1) for the production of plastics objects (2) each having at least one rod-shaped metal insert (3) which has a proximal end (12) and a distal end (12a) having a ground surface, in particular medical piercing means, in an injection moulding process, comprising a first mould half element (4) and a second mould half element (5), it being possible to shift at least one of the mould half elements (4, 5) in a closing direction (Z) in such a way that the two mould half elements (4, 5) together form a closed cavity (7) in a closed position (6b) for moulding a plastics object (2), the insert (3), in order to be overmoulded with plastics material, being arranged so as to project into the cavity (7) at least in portions and being arranged substantially between the two mould half elements (4, 5) in a mould separation plane (8) which comprises a longitudinal direction (X) and a width direction (Y),
**characterised in that**
at least one of the mould half elements (4, 5) has at least one fixing means (9, 10) by means of which the insert (3) can be fixed in the width direction (Y), and the one positioning means (11) can be arranged at the proximal end (12) of the insert (3) and can be used to shift the insert (3), fixed in the width direction (Y), in the longitudinal direction (X) towards an injection position (13).

2. Device (1) according to claim 1,
**characterised in that**
the positioning means (11) comprises a sliding core (14) which is arranged substantially in the mould separation plane (8), the sliding core (14) having an end face (15) by means of which an end face (16) at the proximal end (12) of the insert (3) can be contacted, as a result of which a translational force can be transmitted to the insert (3) and the insert (3) can be shifted in a longitudinal direction (X) towards the injection position (13).

3. Device (1) according to either claim 1 or claim 2,
**characterised in that**
the positioning means (11) comprises an end piece (17) on which the sliding core (14) is arranged, the mould half elements (4, 5) having recesses (18, 19) which are complementary in portions to the end piece (17) and the end piece (17) being received in the recesses (18, 19) in the closed position (6b) of the mould half elements (4, 5).

4. Device (1) according to claim 3,
**characterised in that**
at least one of the mould halves (4, 5) comprises an actuation surface (20) and the end piece (17) comprises at least one ramp surface (21), it being possible to shift the end piece (17) in a closing movement of the mould half elements (4, 5) from an initial position (22) into an end position (23) by a sliding movement of the at least one actuation surface (20) onto the at least one ramp surface (21).

5. Device (1) according to either claim 3 or claim 4,
**characterised in that**
the device (1) comprises a reset device by means of which the end piece (17), in an opening movement of the mould half elements (4, 5), can be shifted from the end position (23) into the initial position (22).

6. Device (1) according to either claim 4 or claim 5,
**characterised in that**
the injection position (13) of the insert (3) is defined by the end position (23) of the end piece (17), in which position an end face (24) of the end piece (17) contacts the respective end faces (25) of the recesses (18, 19) in the mould half elements (4, 5), and the length (26) of the sliding core (14).

7. Device (1) according to any of the preceding claims,
**characterised in that**
the first mould half element (4) has a first fixing means (9) and the second mould half element (5) has a second fixing means (10), the fixing means (9, 10) leading into the mould separation plane (8), being arranged outside of the cavity (7) and fix the insert (3) in the width direction (Y) of the mould half elements (4, 5).

8. Device (1) according to claim 7,
**characterised in that**
the first fixing means (9) comprises a magnetic portion (27) for fixing the insert (3), the magnetic portion (27) terminating in a planar manner against a surface (28) of the first mould half element (4).

9. Device (1) according to claim 7,
**characterised in that**
the second fixing means (10) comprises at least one centring pin (29), for fixing and centring the insert (3), it being possible to apply pressure to the centring pin (29) by means of a spring element (30) such that said pin, in an open position (6a) of the mould half elements (4, 5), projects into the mould separation plane (8) in portions and, in the closed position (6b) of the mould half elements (4, 5), can be shifted out of the mould separation plane (8), counter to the closing direction (Z), against the spring force.

10. Device (1) according to claim 9,
**characterised in that**
the centring pin (29) has centring surfaces (33) on its end face (32) for centring the insert (3) with respect to the width direction (Y).

11. Device (1) according to any of the preceding claims,
**characterised in that**
the device (1) contains at least one runner (34) which leads to the cavity (7) and is guided at least in part in the second mould half element (5).

12. Device (1) according to claim 11,
**characterised in that**
a preferably trapezoidal sliding core cover (35) is arranged between the sliding core (14) and the runner (34) in the region of the at least one runner (34).

13. Device (1) according to any of the preceding claims,
**characterised in that**
the first (4) and the second mould half element (5) are arranged horizontally, the second mould half element (5) being arranged above the first mould half element (4) in the vertical closing direction (Z).

## Revendications

1. Dispositif (1) de fabrication de pièces en matière plastique (2) comportant chacune au moins un insert métallique (3) en forme de tige, lequel présente une extrémité proximale (12) et une extrémité distale (12a) avec un tranchant, en particulier de moyens de piquage médicaux, dans un procédé de moulage par injection, comportant un premier élément demi-moule (4) et un second élément demi-moule (5), au moins l'un des éléments demi-moules (4, 5) étant déplaçable le long d'une direction de fermeture (Z) de telle sorte que les deux éléments demi-moules (4, 5) dans une position de fermeture (6b) forment ensemble une cavité fermée (7) pour le moulage d'une pièce en matière plastique (2), l'insert (3), pour le surmoulage avec la matière plastique, étant disposé se projetant au moins par parties dans la cavité (7) et sensiblement entre les deux éléments demi-moules (4, 5) dans un plan (8) de division de moule, lequel comporte une direction longitudinale (X) et une direction de la largeur (Y),
**caractérisé par le fait que**
au moins l'un des éléments demi-moules (4, 5) présente au moins un moyen de fixation (9, 10), avec lequel l'insert (3) est apte à être fixé dans la direction de la largeur (Y), et **par le fait qu'**un moyen de positionnement (11) est apte à être disposé à l'extrémité proximale (12) de l'insert (3), au moyen duquel l'insert (3), qui est fixé dans la direction de la largeur (Y), est apte à être déplacé le long de la direction longitudinale (X) jusque dans une position d'injection (13).

2. Dispositif (1) selon la revendication 1,
**caractérisé par le fait que**
le moyen de positionnement (11) comporte un noyau coulissant (14), lequel est disposé sensiblement dans le plan (8) de division de moule, le noyau coulissant (14) présentant une surface frontale (15), qui est apte à venir en contact avec une surface frontale (16) à l'extrémité proximale (12) de l'insert (3), ce par quoi une force de translation est apte à être transférée à l'insert (3) et l'insert (3) est apte à être déplacé jusque dans la position d'injection (13) le long d'une direction longitudinale (X).

3. Dispositif (1) selon l'une des revendications 1 ou 2,
**caractérisé par le fait que**
le moyen de positionnement (11) comporte une pièce d'extrémité (17) sur laquelle est disposé le noyau coulissant (14), les éléments demi-moules (4, 5) présentant des logements (18, 19), qui sont formés par parties complémentaires de la pièce d'extrémité (17), et, dans la position de fermeture (6b) des éléments demi-moules (4, 5), la pièce d'extrémité (17) étant reçue dans les logements (18, 19).

4. Dispositif (1) selon la revendication 3,
**caractérisé par le fait que**
au moins l'un des demi-moules (4, 5) comporte une surface d'actionnement (20) et la pièce d'extrémité (17) comporte au moins une surface en rampe (21), la pièce d'extrémité (17) étant apte à être déplacée lors d'un mouvement de fermeture des éléments demi-moules (4, 5) d'une position de départ (22) jusque dans une position de fin (23) par un mouvement coulissant de ladite au moins une surface d'actionnement (20) sur ladite au moins une surface en rampe (21).

5. Dispositif (1) selon l'une des revendications 3 ou 4,
**caractérisé par le fait que**
le dispositif (1) comporte un dispositif de rappel, au moyen duquel la pièce d'extrémité (17) est apte à être déplacée de la position de fin (23) jusque dans la position de départ (22) lors d'un mouvement d'ouverture des éléments demi-moules (4, 5).

6. Dispositif (1) selon l'une des revendications 4 ou 5,
**caractérisé par le fait que**
la position d'injection (13) de l'insert (3) est établie par la position de fin (23) de la pièce d'extrémité (17), dans laquelle une surface frontale (24) de la pièce d'extrémité (17) vient en contact avec les surfaces frontales respectives (25) des logements (18, 19) des éléments demi-moules (4, 5), et par la longueur (26) du noyau coulissant (14).

7. Dispositif (1) selon l'une des revendications précédentes,
**caractérisé par le fait que**
le premier élément demi-moule (4) présente un premier moyen de fixation (9) et le second élément demi-moule (5) présente un second moyen de fixation (10), les moyens de fixation (9, 10) débouchant dans le plan (8) de division de moule, étant disposés à l'extérieur de la cavité (7) et fixant l'insert (3) dans la direction de la largeur (Y) des éléments demi-moules (4, 5).

8. Dispositif (1) selon la revendication 7,
**caractérisé par le fait que**
le premier moyen de fixation (9) comporte une partie magnétique (27) pour la fixation de l'insert (3), la partie magnétique (27) se terminant à plat avec une surface (28) du premier élément demi-moule (4).

9. Dispositif (1) selon la revendication 7,
**caractérisé par le fait que**
le second moyen de fixation (10) comporte au moins une broche de centrage (29), pour la fixation et le centrage de l'insert (3), la broche de centrage (29) étant mise en pression par un élément à ressort (30), de telle sorte que, dans une position ouverte (6a) des éléments demi-moules (4, 5), elle se projette par parties dans le plan (8) de division de moule et dans la position de fermeture (6b) des éléments demi-moules (4, 5), elle est apte à être déplacée, à l'encontre de la force de ressort, hors du plan (8) de division de moule à l'encontre de la direction de fermeture (Z).

10. Dispositif (1) selon la revendication 9,
**caractérisé par le fait que**
la broche de centrage (29) présente des surfaces de centrage (33) sur son côté frontal (32), afin de centrer l'insert (3) par rapport à la direction de la largeur (Y).

11. Dispositif (1) selon l'une des revendications précédentes,
**caractérisé par le fait que**
le dispositif (1) contient au moins un canal d'injection conduisant à la cavité (7), lequel est guidé au moins partiellement dans le second élément demi-moule (5).

12. Dispositif (1) selon la revendication 11,
**caractérisé par le fait que**
un couvercle (35) de noyau coulissant, de forme de préférence trapézoïdale, est disposé dans la zone dudit au moins un canal d'injection (34) entre le noyau coulissant (14) et le canal d'injection (34).

13. Dispositif (1) selon l'une des revendications précédentes,
**caractérisé par le fait que**
le premier (4) et le second (5) élément demi-moule sont disposés horizontalement, le second élément demi-moule (5) étant disposé au-dessus du premier élément demi-moule (4) dans la direction de fermeture (Z) verticale.
